(19) Europäisches Patentamt
European Patent Office
Office européen des brevets

(11) Publication number: 0 254 431 A2

# (12) EUROPEAN PATENT APPLICATION

(21) Application number: 87305679.0

(51) Int. Cl.4: C07C 65/24 , C08G 61/12

(22) Date of filing: 25.06.87

(30) Priority: 26.06.86 US 878610

(43) Date of publication of application: 27.01.88 Bulletin 88/04

(84) Designated Contracting States: DE FR GB NL

(71) Applicant: E.I. DU PONT DE NEMOURS AND COMPANY
1007 Market Street
Wilmington Delaware 19898(US)

(72) Inventor: Sweeney,Wilfred
15 S. Cliffe Drive
Wilmington Delaware 19809(US)

(74) Representative: Woodman, Derek et al
FRANK B. DEHN & CO. Imperial House 15-19 Kingsway
London WC2B 6UZ(GB)

## (54) Polyether ketone.

(57) A novel poly(etherketone) is made from 4-(2,6-dimethylphenoxy) benzoic acid. Tough films are formed from the polymer.

EP 0 254 431 A2

# Polyether Ketone

## Background of the Invention

Aromatic polyetherketones are known to have high glass transition temperatures and excellent thermal and hydrolytic stabilities. Some have been commercialized for high temperature composite uses. Synthesis of the product has been based on a relatively expensive condensation of a bis(halophenyl)ketone at high temperatures with alkali metal salts of a diphenol. The product is then purified by removal of solvent and the salt byproduct.

A simpler route to aromatic polyetherketones involves Friedel-Crafts condensation of an acid halide with an electron-rich aromatic ring. This is indicated below.

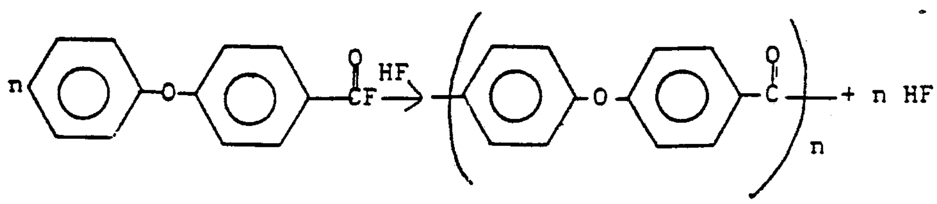


A serious drawback of this general route is that acylation is not limited to the para position but occurs also in positions ortho to the activating oxygen group which may result in branching and coloration. An object of this invention is to provide polyetherketones and precursors therefor which are not subject to these deficiencies.

## Summary of the Invention

This invention provides a novel film-forming polyether ketone having repeating units of the formula

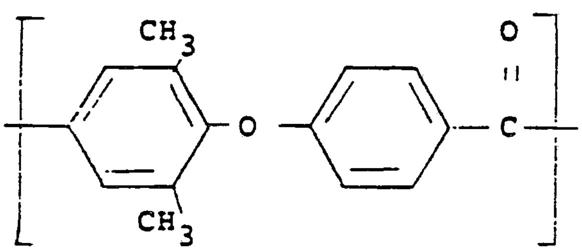

and formed by a Friedel-Crafts polycondensation of 4-(2,6-dimethylphenoxy) benzoic acid.

## Detailed Description of the Invention

This application relates to the poly(etherketone) derived from 4-(2,6-dimethylphenoxy)benzoic acid. The polymer is formed by an internal Friedel-Crafts acylation in the presence of a Lewis acid. The polymer is colorless and completely free from gel.

Tough films may be made from the polymer by conventional melt or solvent casting and can be oriented by drawing to increase strength and modulus. The polymer may be used in composites or in membranes for gas or salt separation.

4-(2,6-Dimethylphenoxy) benzoic acid is prepared by reacting a 4-fluorobenzoic acid ester with 2,6-dimethyl phenol or an alkali metal salt thereof. Alkyl esters of 4-fluorobenzoic acid may be employed and ethyl p-fluorobenzoate is preferred. Potassium 2,6-dimethyl-phenolate is particularly preferred for this condensation reaction.

The reaction conditions are well-known in the art. A solvent such as dimethyl sulfoxide is employed for the reaction medium. An activated copper catalyst (Organic Synthesis, Vol. II, p. 446) and potassium fluoride are added and the reaction is carried out to completion by heating in an inert atmosphere. The resulting ester is hydrolyzed to form 4-(2,6-dimethyl-phenoxy) benzoic acid.

Preparation of the poly(etherketone) is achieved by reacting the above acid with a Lewis acid in a conventional Friedel-Crafts acylation. The inherent viscosity of the film-forming polymer should be at least 0.5 dL/g (deciliters/gram). The inherent viscosity determination is made at a concentration of 0.5% by wt. polymer in 98% sulfuric acid at 30°C.

The following example is intended to illustrate the present invention and should not be interpreted as limiting.

Example I

4-(2,6-Dimethylphenoxy) benzoic acid was prepared by condensing dry potassium 2,6-dimethylphenolate with ethyl p-fluorobenzoate in dimethyl sulfoxide containing activated copper catalyst and potassium fluoride by heating under nitrogen at 95-100°C for 48 hours followed by 3 hours at 120°C. The solvent was removed under ca. 50 mm vacuum and the residue mixed with water. The mixture was extracted with methylene chloride. After washing with water and drying over calcium chloride, the methylene chloride was removed and the residue distilled. The fraction boiling at 132-138°C was essentially pure ethyl 4-(2,6-dimethylphenoxy)benzoate.

63 g of the ester was hydrolyzed by refluxing with 30 g sodium hydroxide in 400 cc water containing 10 drops of Triton X-100 to give a clear solution. Acidification to a pH of I with sulphuric acid gave the phenoxy acid as a cream colored precipitate with a melting point of 154-155°C. Elemental analysis and proton NMR confirmed the expected structure.

20 g of the above acid was heated with agitation in a stainless steel autoclave with 100 g hydrogen fluoride and 25 g boron trifluoride for 3 hours at 80-100°C. The volatiles were vented and a tough off-white polymer was isolated by triturating with ice and washing in a blender with aqueous sodium bicarbonate to remove acidic residues. The polymer was soaked overnight in methanol, dissolved completely in trifluoroacetic acid from which it was precipitated into water to yield a colorless fibrous polymer. The polymer had an inherent viscosity of 1.29 dL/g. After washing well with acetone the polymer was dried overnight at 60°C under vacuum. X-ray examination showed the resulting polymer to be essentially amorphous. Tough films could be pressed at 350°C and 1500 psi or, alternatively, solvent cast from chloroform. These films could be drawn 3-4 X at 200°C to yield the following tensile properties--tenacity/elongation/modulus of I gpd/4%/32 gpd (20 kpsi/4%/600 kpsi). The polymer was thermally very stable and showed no weight loss on heating in nitrogen to 470°C.

Example 2

Comparative Example

The above polymerization was simulated by polymerizing 25 g of 3-carboxyphenyl ether with 100 g hydrogen fluoride and 25 g boron trifluoride in an autoclave for 3 hours at 80-100°C. A tough red polymer resulted. This was isolated by fibrillating in a blender with excess water, filtered and washed repeatedly to remove residual acid. The polymer was then washed with acetone and vacuum dried. The polymer was then dissolved in 50/50 (v/v) chloroform/trifluoroacetic acid and filtered through glass wool to remove gel. The solution was then added to excess ethanol in a blender to yield a pink fibrous polymer. After drying at 60°C in vacuum under nitrogen, tough films were pressed at 250°C. These were deep red in color in contrast to those from the polymer in Example I.

Example 3

Comparative Example

Dibenzofuran is a cyclic analog of phenyl ether in which one of the electron rich ortho positions in each ring is part of the ring system and hence is unavailable for electrophilic attack. Thus, each benzene ring contains one ortho and one para position available for electrophilic condensation. I6.8 g of distilled dibenzofuran, 20.3 g distilled isophthaloyl chloride, I00 g orthodichlorobenzene were heated at 50°C for 3 hours in an autoclave with I00 g hydrogen fluoride and 25 g boron trifluoride. The polymer was isolated by washing in a blender with water and repeatedly with I0% aqueous sodium carbonate till neutral and again with water. After drying the resulting polymer melted at about 280°C and yielded deep red brittle films.

## Claims

I. 4-(2,6-Dimethylphenoxy)benzoic acid.

2. A poly(etherketone) having repeating units of the formula

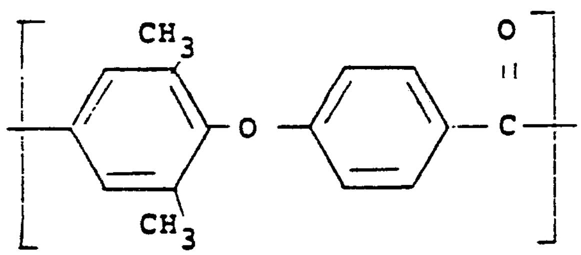

and an inherent viscosity of at least 0.5 dL/g.

3. A process for preparing the polymer claimed in claim 2 comprising heating 4-(2,6-dimethylphenoxy)-benzoic acid in the presence of a Lewis acid.

4. A process as claimed in claim 3 wherein the catalyst is a combination of hydrogen fluoride and boron trifluoride.

5. A film of the polymer claimed in claim 2.

6. Use of the polymer claimed in claim 2 in composites or in membranes for gas or salt separation.

7. A process for the preparation of 4-(2,6-dimethylphenoxy)benzoic acid which comprises reacting an ester of 4-fluorobenzoic acid with 2,6-dimethylphenol or with an alkali metal salt thereof, and subsequently hydrolyzing the ester functionality of the compound thereby obtained.

8. A process as claimed in claim 7 wherein the ester of 4-fluorobenzoic acid used is ethyl p-fluorobenzoate.

9. A process as claimed in claim 7 or claim 8 wherein the alkali metal salt of 2,6-dimethylphenol used is potassium 2,6-dimethylphenolate.